# EUROPEAN PATENT APPLICATION

(11) **EP 1 977 773 A1**
(43) Date of publication of application: **08.10.2008**
(21) Application number: 07707220.5
(22) Date of filing: 23.01.2007
(51) Int. Cl.: A61M 1/14, A61K 31/194, A61P 7/08

(54) **AMELIORATING AGENT FOR ALUMINUM STORAGE DISEASE**

(30) Priority: 24.01.2006 JP 2006014904
(71) Applicant: Ajinomoto Co., Inc., Tokyo 104-8315 (JP)
(72) Inventor: KOIZUMI, Tomonori, Kawasaki-shi Kanagawa 210-8681 (JP); ISAKA, Mitsuyoshi, Kawasaki-shi Kanagawa 210-8681 (JP); SHIRAI, Yoshiaki, Kawasaki-shi Kanagawa 210-8681 (JP); MIHARA, Ryuuichi, Kawasaki-shi Kanagawa 210-8681 (JP); UBUKATA, Kazuyuki, Kawasaki-shi Kanagawa 210-8681 (JP); NAKAYAMA, Satoshi, Kawasaki-shi Kanagawa 210-8681 (JP)
(74) Representative: Nicholls, Kathryn Margaret
(86) International application number: PCT/JP2007/050957
(87) International publication number: WO 2007/086361

(57) **Abstract**

A safe and easy-handling method to eliminate aluminum, which can easily accumulate in a dialysis patient body, in the course of a dialysis treatment and a dialysate used in the above method to eliminate aluminum ions from blood and tissues in the body are provided. A method to eliminate aluminum from a dialysis patient body **characterized in that** a dialysate mixed with aluminum complexing agents allows formation of complexes with aluminum to excrete aluminum in the form of aluminum complexes into the dialysis drain fluid is also provided. More specifically, the above-mentioned method to eliminate aluminum from the body employs preferably citric acid as an aluminum complexing agent, and the dialysate comprises general dialysate components and 1.7-2.4 mEq/L of citric acid as an aluminum complexing agent.

## Description

### TECHNICAL FIELD

The present invention relates to methods to accelerate elimination of aluminum from the body by hemodialysis treatment, and more particularly, to a method to accelerate elimination of aluminum from the body into the dialysis drain fluid in the form of aluminum complexes by mixing aluminum complexing agents in dialysates to allow formation of complexes with aluminum combined with blood plasma proteins or existing in the tissues of a dialysis patient body.

### BACKGROUND ART

Most of aluminum taken in a human body derives from food and food additives, or otherwise, from drinking water, cooking utensils made of aluminum and so on. The amount of aluminum taken per day varies region by region and dietary habit by habit. WHO (World Health Organization) reports that the daily intake amounts to 2.5-13 mg.

According to surveys by WHO and FAO (Food and Agriculture Organization) regarding effects of aluminum on a human body based on the data obtained in a large number of animal studies and others, the amount tolerable to a human body throughout the lifetime without causing no adverse effect (*i.e*. ADI: Acceptable Daily Intake) is set at "7 mg/week × body weight kg". When calculated in a human body weighing 50 kg, the ADI works out at 50 mg per day. Aluminum is said harmless for health as far as taken up to this amount.

Almost all aluminum taken orally is known to be excreted from the body without being absorbed. Although merely about 0.02-0.5% of orally-taken aluminum is absorbed through the intestinal tract into the living body, almost all such absorbed aluminum is immediately excreted into urine as far as kidneys function normally (Non-patent Literature 1, 2). It is said that generally 35-40 mg of aluminum exist in a human body in stable forms mainly found in lungs and bones and slightly in blood and the blain. Actions of aluminum in the body are not known clearly at present.

When it comes to a dialysis patient with kidneys' excreting function disordered, aluminum can easily accumulate in the body because the pathway to excrete aluminum into urine is blocked. Hence, in recent years, such disorders as "aluminum encephalopathy" (i.e. dialysis dementia), one of whose predominant symptom is dementia caused by the aluminum accumulation, and "aluminum osteopathy", whose predominant symptoms are bone cyst and osteomalacia, have been reported in dialysis patients.

Different from Alzheimer-type dementia, aluminum encephalopathy (dialysis dementia) is caused by the neurotoxicity of aluminum excessively taken and absorbed into the body. Some studies on dialysis dementia report that intake of aluminum more than a certain amount can cause neurotoxic symptoms in the patients with kidney failure.
In these circumstances, the Ministry of Health, Labor and Welfare of Japan contraindicates the use of aluminum-containing drugs (such drugs as contain aluminum silicate, aluminum hydroxide gel and magnesium aluminometa silicate) for dialysis patients.

In these days, the quality of tap water used for preparation of dialysates has been improved by the use of water-purification systems employing the reverse osmosis membrane, and the amount of aluminum entering a dialysis patient body has been extremely reduced. However, some dialysis patients are still not free from the risks of aluminum osteopathy or aluminum encephalopathy (dialysis dementia) because of aluminum taken in the past or daily through food, beverages and so on.

For the purpose of eliminating aluminum forcibly from the body, deferoxamine mesilate (DFO), an iron excreting agent, may be injected intravenously once a week at 5 mg/kg. However, the use of DFO is avoided because DFO can cause visual or hearing impairments or other adverse effects. On the other hand, a method to inject aluminum complexing agents in the course of hemodialysis circuit in conventional dialysis treatment was suggested (Patent literature 1). However, no special pharmaceutical agent or medical device was suggested there. This method is unlikely to be used in clinical practice at present because of the complicacy of works required.
[Patent literature 1] Japanese Lid-open Patent Publication No. 6-197958
[Non-patent literature 1] Shozo Nomoto et al., Japanese Journal of Clinical Medicine, 57: 287-289
[Non-patent literature 2] Fennington et al., Food Addit. Contam., 12: 119-128

### DISCLOSURE OF THE INVENTION

### PROBLEMS TO BE SOLVED BY THE INVENTION

In the view of the above problems, the present invention has as an object to provide a safe and easy-handling method to eliminate aluminum, which can easily accumulate in a dialysis patient body, in the course of dialysis treatment.
The present invention also has as another object to provide a dialysis preparation that is used in the above method for the purpose of eliminating aluminum ions existing in blood and tissues in the body.

### MEANS FOR SOLVING THE PROBLEMS

A basic embodiment of the invention is a dialysis fluid (dialysate) that includes aluminum complexing agents.

More specifically, the above-mentioned dialysis preparation includes the aluminum complexing agents selected from citric acid, L-glutamate diacetate, and L-aspartic acid diacetate.

Another embodiment of the invention is a method to eliminate aluminum from a dialysis patient body characterized in that a dialysate comprising aluminum complexing agents allows the formation of aluminum complexes and such formed aluminum complexes are excreted into the dialysis drain fluid. More specifically, the above-mentioned method to eliminate aluminum from the body employs the aluminum complexing agents selected from citric acid, L-glutamate diacetate, and L-aspartic acid diacetate.

### EFFECTS OF THE INVENTION

A dialysis preparation for aluminum elimination provided by the invention eliminates aluminum from a dialysis patient body in the course of a dialysis treatment by a method that aluminum complexing agents mixed in a dialysate combine with aluminum in the body to form aluminum complexes and such formed aluminum complexes are excreted into the dialysis drain fluid. Therefore, the present invention reduces the risks of occurrences of aluminum osteopathy or aluminum encephalopathy (dialysis dementia).

Further, a dialysate provided by the present invention is not risky from the viewpoints of safety to dialysis patients because such dialysate is assumed to be used with antecedent confirmations of causing no adverse effect non-clinically and clinically. Another advantage of the present invention is the easiness of handling because medical workers are required to do no additional work than substitute a dialysate provided by the present invention for a conventional dialysate in the scene of dialysis treatment.

### BEST MODE FOR CARRYING OUT THE INVENTION

As described above, the present invention provides a dialysate mixed with aluminum complexing agents to drain aluminum, which can easily accumulate in a dialysis patient body, into the dialysis drain fluid. The present invention is characterized by the safeness and easiness in handling of the above-mentioned aluminum-eliminating method.

Basically a dialysate used for the present invention is an existing dialysate whose safety was confirmed in human, whereto aluminum complexing agents are added.

Specifically, the dialysate comprises as general dialysate components 130-140 mEq/L of sodium, 2-2.5 mEq/L of potassium, 2.5-3.75 mEq/L of calcium, 1-1.5 mEq/L of magnesium, 104-115 mEq/L of chlorine, 8-38 mEq/L of acetic acid, at most 35 mEq/l of bicarbonate and at most 200 mEq/L of glucose.

In accordance with the present invention, a suitable dialysate comprising the above-mentioned components is selected to add aluminum complexing agents; then, a pH level of the dialysate and concentrations of calcium, magnesium or any other components thereof that can affect the formation of aluminum complexes are controlled to produce a dialysate that has good dialysis effects as well as aluminum-elimination effects.

Any aluminum complexing agent can be used in the present invention with the proviso that its safety and essential effectiveness as a dialysate in dialysis patients are confirmed. Specifically, suitable aluminum complexing agents include citric acid, L-glutamate diacetate, and L-aspartic acid diacetate. Especially, a preferable aluminum complexing agent is citric acid.

When citric acid is used as the aluminum complexing agent, preferably the concentration of citric acid is between 1.7 and 2.4 mEq/L. The concentration below 1.7 mEq/L produces less significant aluminum-eliminating effect. The concentration exceeding 2.4 mEq/L produces little additional effect compared to the range between 1.7 and 2.4 mEq/L and may damage the functionality of dialysate.

The present invention has been described with respect to adding aluminum complexing agents to a dialysate for eliminating aluminum from a dialysis patient body. However, the present invention is also applicable to eliminating any other metal ions than aluminum that are not essential for the living body and cause undesirable effects in the body.
Thus, it should be appreciated that the utilization of a dialysate provided by the present invention for this purpose is included in the scope of the present invention.

### EXAMPLES

By way of test examples, where specific efficacies were confirmed, and working examples, and not limitation, detailed description of the invention will be set forth.

The present invention provides a dialysate comprising aluminum complexing agents. Preferably, the aluminum complexing agent is citric acid. In the following examples, the test results indicate the effectiveness of the dialysate comprising citric acid as an aluminum complexing agent in the elimination of aluminum from the body. Meanwhile, it had been confirmed that, when 1. 7 mEq/L of citric acid had been added to a dialysate during a dialysis treatment, the citric acid concentration in the patient blood plasma had risen by about 3 mg/dL. In the following examples, how this phenomenon effects on the aluminum kinetics *in vivo* was examined.

### Test Example 1:

Whether aluminum combined to proteins chelates with citric acid and is dissociated from the proteins in rat plasma was examined.
A used indicator was the protein-binding rates (by ultrafiltration method) when citric acid was added and when not added.

### <Experimental means>

Aluminum was added to rat blood plasma to make the concentration 50 µg/L, and citric acid was added there to make the final concentration rise by 3 mg/dL. Then, it was incubated at 37 °C for 60 minutes. After that, the solution was centrifuged in a centrifuge (Centrifree) to obtain about 100 µL of filtrate. The aluminum concentrations of the added sample, the supernatant and the filtrate were measured respectively by the HPLC method (Chromatoracer Al; SHINO-TEST Corp.) to calculate the protein-binding rates.

### <Results>

The free-aluminum percentage was 35.5 ± 0.4% in the plasma with citric acid added as an aluminum complexing agent, higher against 23.4 ± 10.0% in the plasma with no citric acid added.
From the above, confirmed was the efficacy that citric acid as an aluminum complexing agent forms complexes between citric acid and aluminum combined with proteins in the blood plasma to disassociate aluminum from the proteins.

### Test Example 2:

After aluminum accumulations were made in rat tissues, citric acid as an aluminum complexing agent was administered intravenously repeatedly to examine whether citric acid would eliminate the aluminum accumulated in the tissues.

### <Experimental means>

Aluminum was administered to male rats intraperitoneally repeatedly once a day for 6 weeks at 2 mg/kg. Then, the rats were cannulated in the jugular vein.
From the jugular cannula, 0.75% citric acid solution was administered repeatedly taking the same dialysis time and frequency as in human; that is, for 4 hours continuously intravenously three times a week for 4 weeks.
For the control group, physiological saline was administered in the same means. Each group had 4 to 6 rats.
After the 4-week administration, thigh bones were collected from the rats to compare the aluminum levels in the thigh bones of both groups using the ratios of the aluminum peak area analyzed by the HPLC method against the peak area of internal standard substance as an indicator.

### <Results>

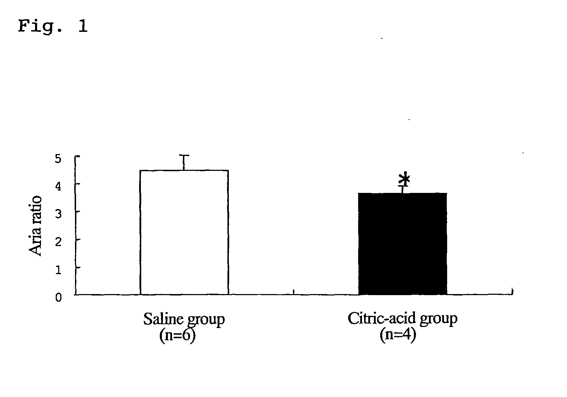

The examination results are shown in the figure 1. The area ratios of the control group against the citric acid administered group were respectively 4.46 ± 0.52 against 3.62 ± 0.27, which indicates that the thighbone aluminum levels of citric acid administered group were statistically significantly lower than those of the control group.
From the above, confirmed was the efficacy that the dialysis with a dialysate comprising citric acid as an aluminum complexing agent eliminated the aluminum accumulated in the bone.

### Working examples:

Dialysates were prepared in the following prescriptions.

**Table 1:**

| Components | Example 1 | Example 2 |
|---|---|---|
| Na⁺ | 140 mEq/L | 140 mEq/L |
| K⁺ | 2.0 mEq/L | 2.0 mEq/L |
| Ca⁺ | 3.0 mEq/L | 3.0 mEq/L |
| Mg²⁺ | 1.0 mEq/L | 1.0 mEq/L |
| Cl⁺ | 111 mEq/L | 111 mEq/L |
| HCO3⁻ | 35 mEq/L | 1.5 mEq/L |
| Glucose | 1.5 g/L | 1.5 g/L |
| Citric acid (citric acid and sodium citrate) | 1.7 mEq/L | 2.4 mEq/L |

### INDUSTRIAL APPLICABILITY

As described above, according to the present invention, aluminum is eliminated from a dialysis patient body in the course of a dialysis treatment by a method that aluminum complexing agents mixed in a dialysate allow formation of complexes between the aluminum complexing agents and aluminum in the body and such formed complexes are excreted into the dialysis drain fluid. Thus, the present invention has great medical effectiveness in reduction of risks of aluminum osteopathy or aluminum encephalopathy (dialysis dementia) occurrences in dialysis patients.

### BRIEF DESCRIPTION OF DRAWINGS

Figure 1 shows the results of Test Example 1. Values are expresses as means ± S.D. Single asterisk indicates P<0.05 analyzed by Student's t-test.

## Claims

1. A dialysate comprising aluminum complexing agents.

2. The dialysate of claim 1 wherein the aluminum complexing agents are selected from citric acid, L-glutamate diacetate, and L-aspartic acid diacetate.

3. A method to eliminate aluminum from a dialysis patient body **characterized in that** a dialysate mixed with aluminum complexing agents allows formation of aluminum complexes and such formed complexes are excreted into the dialysis drain fluid.

4. The method of claim 3 wherein the aluminum complexing agents are selected from citric acid, L-glutamate diacetate, and L-aspartic acid diacetate.
